# EUROPÄISCHE PATENTSCHRIFT

(11) **EP 3 153 192 B1**
(45) Veröffentlichungstag und Bekanntmachung des Hinweises auf die Patenterteilung: **12.12.2018**
(21) Anmeldenummer: 16192615.9
(22) Anmeldetag: 06.10.2016
(51) Int. Cl.: A61M 1/14, E05B 47/00

(54) **MEDIZINISCHES GERÄT MIT VERRIEGELUNGSZUSTAND-ERFASSUNGSVORRICHTUNG**
MEDICAL DEVICE WITH LOCK STATE DETECTION DEVICE
APPAREIL MÉDICAL AVEC DISPOSITIF DE DÉTECTION D'ÉTAT DE VERROUILLAGE

(30) Priorität: 07.10.2015 DE 102015117095
(43) Veröffentlichungstag der Anmeldung: 12.04.2017
(73) Patentinhaber: B. Braun Avitum AG, 34212 Melsungen (DE)
(72) Erfinder: Iske, Andreas, 34320 Söhrewald (DE)
(74) Vertreter: Winter, Brandl, Fürniss, Hübner, Röss, Kaiser, Polte - Partnerschaft mbB

(56) Entgegenhaltungen:
- WO-A2-2013/067359
- DE-A1-102009 022 838
- DE-A1-102012 111 342
- DE-U1-202005 013 065
- US-A1- 2007 024 442

## Beschreibung

Die Erfindung betrifft eine Verriegelungszustand-Erfassungsvorrichtung, und bezieht sich insbesondere auf eine Verriegelungszustand-Erfassungsvorrichtung in Form einer Verriegelungsvorrichtung für Türen oder Klappen mit einer Riegelerkennung zur Verwendung vorwiegend bei Blutbehandlungseinrichtungen, wie beispielsweise Dialysegeräten.

Bei beispielsweise extrakorporalen Blutbehandlungseinrichtungen, etwa Dialysegeräten zur Durchführung einer Hämodialysebehandlung, werden zum Schutz vor Verbrennungen des Benutzers bei Berühren und zum Erkennen und/oder Auffangen von Leckagen die bei Einrichtungen dieser Art üblicherweise vorgesehenen Dialysierflüssigkeitsfilter mit einer Gerätetür geschützt.

Geräte, die über Erkennungseinrichtungen zum Erkennen einer geschlossenen Tür verfügen, sind bekannt. In der Regel ist dabei eine Schaltkomponente beweglich an beispielsweise an einer Tür und eine weitere Schaltkomponente als feststehendes Gegenstück beispielsweise am Gehäuse befestigt.

Beispielsweise offenbart die WO 2013 67359 A2 einen an einer hakenartigen Einrichtung an einer Tür befestigten, beweglichen Magneten und einen an einem Gehäuse angeordneten und dort feststehenden Sensor zur Erfassung von dessen Magnetfeld, wenn sich der Magnet zu dem Sensor hin bewegt. Aufgrund der Art der beschriebenen Erfassung mittels Hallsensoren, die für die erforderliche Schaltgenauigkeit benötigt werden, kann hierbei das Magnetfeld des Magneten den Sensor bei Annäherung bereits im geöffneten Zustand schalten. Hallsensoren sind teuer und komplex in der Anwendung, sie benötigen eine Spannungsversorgung, und die Schaltungsanordnung insgesamt ist temperaturanfällig.

Ferner zeigt die US 2002 0 165 503 A1 eine mittels eines Hakens arbeitende Anordnung, die einen Sender und einen Empfänger als elektronische Bauteile voraussetzt. Eine ebenfalls erforderliche Reflexionsfläche für die Sensorik des Hakens kann im Laufe der Zeit, beispielsweise aufgrund von Alterung oder Verschmutzung, ihre Reflexionsfähigkeit verlieren, was mit einer Einschränkung oder gar einem Verlust der Funktion einhergeht. Ferner benötigen die eingesetzten Baugruppen ebenfalls eine Versorgungsspannung, ist eine sehr genaue Abstimmung erforderlich und ist die Anordnung in sich lichtanfällig.

Darüber hinaus zeigt DE 20 2005 013 065 U1 eine Vorrichtung zur Positionserkennung mit einem Magneten, einer Reedplatine und einem Abdeckblech, welches in den Raum zwischen dem Magneten und der Reedplatine bewegbar ist und das Magnetfeld beeinflusst.

US 2007/0024441 A1 offenbart ein System zur Positionserkennung eines Riegels von einem Sicherheits-Rolltor, an dessen Schienen ein Magnet und ein Reed-Schalter so angebracht sind, dass sie sich an einer Bolzenöffnung an einer Schiene gegenüberliegen. Der Riegel ist magnetisch und wird bei Verriegelung des Rollladens durch Drehen eines Verriegelungsgriffs in die Bolzenöffnung ausgefahren und beeinflusst dadurch das Magnetfeld zwischen Reed-Schalter und Magnet.

DE 10 2009 022 838 A1 beschreibt ein Kühl- und/oder Gefriergerät mit einem Korpus und einer Tür, wobei am Korpus oder der Tür jeweils ein Magnet und ein Sensor relativ zueinander fest angeordnet sind und ein Abschirmblech durch Bewegung das Magnetfeld im Bereich des Sensors beeinflusst.

Wie vorstehend anhand der Beispiele des Standes der Technik WO 2013 67359 A2 und US 2002 0 165 503 A1 beschrieben wurde, erkennen bekannte Anordnungen zwar geschlossene Türen oder Klappen. Jedoch werden nicht vollständig verschlossene Türen aufgrund von Schalthysteresen der verwendeten Sensoren nicht immer erkannt. Solche nicht vollständig verschlossenen Türen stehen dann einen Spaltbreit offen. Das Gerät erkennt jedoch dennoch und fälschlicherweise eine geschlossene Tür. Sicherheitsfunktionen, wie zum Beispiel eine Erkennung einer Leckage bzw. eines Flüssigkeitsverlusts, können dann funktionslos sein. Die bekannten Anordnungen verwenden ferner teilweise aufwendige Schaltungen (Hallsensor), um eine höhere Genauigkeit zu erzielen. Die aufwendigen Schaltungen benötigen jedoch eine Versorgungsspannung und sind zudem sehr temperaturanfällig. Andere Schaltungen wiederum erfordern einen aktiven Sender und einen aktiven Empfänger. Häufig kommen auch Mikroschalter zur Anwendung. Diese haben den Nachteil, dass die mechanischen Komponenten immer vor Feuchtigkeit geschützt werden müssen. Außerdem reißen die Membranen der Mikroschalter im Laufe der Zeit häufig ein und führen dann zum Ausfall des Schalters.
Bisher erkennen bekannte Erkennungseinrichtungen, oder Erkennungssysteme, jedoch nicht, ob eine geschlossene Tür tatsächlich verriegelt oder beispielsweise nur angelehnt ist. Lediglich angelehnte Türen können beispielsweise Geräteschutzmaßnahmen, wie etwa eine Leckageüberwachung, außer Kraft setzen. In anderen Worten erkennen bekannte Einrichtungen und Systeme, insbesondere auf dem Gebiet der Blutbehandlung, bislang nicht den tatsächlichen Zustand einer Verriegelung.

Der Erfindung liegt daher als eine Aufgabe zugrunde, ein medizinisches Gerät zu schaffen, das eine Verriegelungszustand-Erfassungsvorrichtung bzw. eine Verriegelungsvorrichtung mit einer Riegelerkennung aufweist, mit welcher ein tatsächlich verriegelter Zustand einer Gerätetür oder Geräteklappe, insbesondere an einer Blutbehandlungsvorrichtung, sicher erfassbar ist.

Erfindungsgemäß wird diese Aufgabe durch ein Gerät mit den in Anspruch 1 angegebenen Merkmalen gelöst. Vorteilhafte Weiterbildungen der Erfindung sind Gegenstand der beigefügten Unteransprüche
Gemäß einer grundlegenden Idee der Erfindung wird für beispielsweise Dialysierflüssigkeitsfilter in Blutbehandlungsanlagen, die dazu vorgesehen sind, die Qualität der Dialysierflüssigkeit zu verbessern und die zum Schutz vor Verbrennungen des Anwenders, Benutzers und/oder Patienten durch Berührung und zum Erkennen (vermittels einer Überwachung und beispielsweise durch einen Leckagesensor) bzw. Auffangen von Leckagen mit einer Gerätetür geschützt sind, eine Zustandserkennung für diese Tür bereitgestellt. Damit die bereitgestellten Schutzvorrichtungen sicher funktionieren können, muss das Gerät erkennen können, ob bzw. dass das Gehäuse verschlossen ist.
Erfindungsgemäß wird dabei nicht eine angelehnte Tür erkannt, sondern es wird erfasst, ob bzw. dass sie richtig verriegelt ist bzw. ein bereitgestellter Verschlussriegel ebenfalls geschlossen ist, d.h. sich in einer vorgesehenen Position befindet und somit die Schutzfunktion des Geräts dauerhaft gegeben ist. Weiter erfindungsgemäß wird eine nur teilweise verschlossene Tür erkannt und auf der Grundlage einer solchen Erkennung der Gerätebetrieb bis zur vollständigen Verriegelung unterbunden. Nur eine vollständig verriegelte Tür erlaubt dem Anwender den Betrieb des Geräts. Die Erkennung ist unanfällig gegenüber Verschmutzungen und Temperaturschwankungen. Die Schaltung ist einfach aufgebaut und benötigt keine aufwendige Ansteuerungs- und Abfragehardware. Ein versehentliches Abziehen von Steckern oder Defekte an Kabeln/Sensoren ist ebenfalls erkennbar.
In anderen Worten überwachen bisherige Lösungen nur den Zustand der Tür über einen Magnetsensor oder einen Mikroschalter. Dabei kann es passieren, dass der Magnetsensor oder der Mikroschalter zu früh schaltet und fälschlicherweise einen sicheren Zustand zurück meldet. Die Erfindung dagegen stellt eine sichere Erkennung der Verriegelung bereit. Ein mitwirkender Sensor als Erfassungseinrichtung erkennt nicht, dass die Tür geschlossen ist, sondern erkennt, dass auch ein Verschlussriegel geschlossen ist. Dies ist nur möglich wenn die Gehäusetür richtig geschlossen ist und sich der Riegel in der richtigen Position befindet. Ein unsicherer Zustand ist somit nicht möglich, denn es wird sicher erkannt, dass eine Tür oder Klappe verschlossen ist und sich diese nicht unbemerkt wieder öffnet. Dafür wird die Position des Verschlussriegels erkannt. Die Erkennung funktioniert nur bei absolut korrekter Stellung des Riegels.

Im Einzelnen sind in einem Gehäuseteil ein Sensor, der auf einen Magneten anspricht, und der zugehörige Magnet verbaut. Zwischen beiden Bauteilen befindet sich ein Luftspalt. Der Sensor wird permanent durch den Magnet geschaltet. Wird das Gehäuse mit einem Deckel bzw. einer Klappe verschlossen, bleibt die Signalausgabe des Sensors unverändert. Erst bei Eintauchen des Riegels, der als solcher Bestandteil der Klappe/Tür bzw. an dieser angeordnet ist, in den Luftspalt zwischen Sensor und Magnet, ändert sich das Signal des Sensors. Vorzugsweise sind dazu sämtliche relevanten Gehäuseteile aus einem nicht ferromagnetischen Werkstoff (beispielsweise Aluminium) hergestellt, und ist der Riegel aus einem ferromagnetischen Werkstoff hergestellt.

Wird die mit der erfindungsgemäßen Vorrichtung versehene Gehäuse/Tür-Kombination verschlossen, verschließt der Riegel die Tür sicher. Zusätzlich unterbricht eine metallische Riegelzunge ein Magnetfeld. Die Unterbrechung des Magnetfelds erfolgt dadurch, dass die Riegelzunge in einen Luftspalt zwischen Sensor und Magnet geführt wird. Die Riegelzunge kann aus unterschiedlichen Werkstoffen gefertigt sein, solange der Werkstoff als solcher ferromagnetische Eigenschaften aufweist. Das Magnetfeld schaltet den Sensor. Auf diese Weise wird sichergestellt, dass die Tür nicht nur geschlossen ist und so Patient und Personal vor Leckagen bzw. Verbrennungen durch heiße Bauteile geschützt sind, sondern auch, dass die Tür verriegelt ist und sich nicht unbeabsichtigt öffnet. Sofern der Riegel nicht vollständig geschlossen wird, ändert der Sensor seinen Status nicht und das Gerät gibt einen Alarm aus. Sofern die Tür nicht in der richtigen Endposition steht, passt der Riegel nicht in den Luftspalt zwischen Magnet und Sensor. Auch in diesem Fall wird das Signal durch den Riegel nicht verändert bzw. beeinflusst. Besonders bevorzugt sind der Sensor und der Magnet jeweils in einem festen Abstand zueinander an ein und demselben Bauteil verbaut. Die als Störkomponente wirkende Riegelzunge ist dagegen immer an einem demgegenüber anderen Bauteil montiert.
Durch die Riegelerkennung kann jeder Zugang zu dem abgesicherten Gerät überwacht werden. Dadurch können beispielsweise Blutdruckmanschetten und/oder dergleichen im Gerät aufgenommen, mit einer Klappe geschützt und mit einem entsprechendem Sensor überwacht werden. Eine Aktivierung von Optionen kann dann über die Riegelerkennung erfolgen. Denkbar ist ebenfalls, anstelle einer Reedsensor/Magnet-Paarung auch eine Hallsensor/Magnet-Paarung anzuordnen, mit dem weiteren Vorteil, dass über eine solche Paarung die Schaltgenauigkeit beeinflussbar ist. Denkbar ist ebenfalls, die Störkomponente (den Riegel) an einem Bauteil feststehend anzuordnen und die Einheit aus Sensor und Magnet zur Störkomponente zu führen, schwenken oder drehen. Der Freiraum, in den die Störkomponente geführt wird, kann bedarfsweise unterschiedliche Formen aufweisen. Denkbar ist ebenfalls, Magnet und Sensor luftspaltfrei auf einer gemeinsamen Seite anzuordnen und die Störkomponente hinreichend an eine so gebildete Magnet-Sensor-Einheit heranführbar zu konfigurieren, um dadurch das Magnetfeld ausreichend umzulenken und/oder abzuschwächen, um einen Schaltwechsel am Sensor auszulösen.

Im Einzelnen wird die Aufgabe gelöst durch ein medizinisches Gerät, das eine Verriegelungszustand-Erfassungsvorrichtung aufweist mit:
einer Magneteinrichtung, die an einem ersten Abschnitt eines Gehäuses angeordnet ist, das eine Öffnung aufweist, die mittels einer Öffnungsverschließeinrichtung verschließbar ist;
einer Erfassungseinrichtung, die an einem zweiten Abschnitt des Gehäuses angeordnet ist, der positionell zu dem ersten Abschnitt derart beabstandet ist, dass eine Luftstrecke zwischen dem ersten und dem zweiten Abschnitt ausgebildet ist; und einer Störkomponente, die an einer in der Öffnungsverschließeinrichtung angeordneten Störkomponentenverschwenkeinrichtung verdrehfest festgelegt und dazu angeordnet ist, mit Drehung der Störkomponentenverschwenkeinrichtung in die Luftstrecke einzuschwenken und aus dieser auszuschwenken,
wobei die Erfassungseinrichtung dazu angeordnet ist, in Zusammenwirkung mit der Magneteinrichtung und der Störkomponente einen verriegelten Zustand und einen nicht verriegelten Zustand der Öffnungsverschließeinrichtung zu erfassen und zur Weiterverarbeitung zu signalisieren.

Bevorzugt ist die Erfassungseinrichtung dazu angeordnet, nur dann, wenn sich die Störkomponente in einer in die Luftstrecke eingeschwenkten Position befindet, einen verriegelten Zustand der Öffnungsverschließeinrichtung zu erfassen und zu signalisieren.

Bevorzugt ist die Öffnungsverschließeinrichtung eine Gerätetür und ist die Erfassungsvorrichtung dazu konfiguriert, eine Zustandserkennung dieser Gerätetür durchzuführen, wobei in einer vorbestimmten Position der Störkomponente ein verriegelter Zustand der Gerätetür erkannt und nur dann der Betrieb des Geräts für den Anwender freigegeben wird.

Bevorzugt ist eine Unterbrechung an Anschlussmitteln, die zumindest einen Stecker und/oder eine Kabelverbindung beinhalten, erfassbar und als entriegelter Zustand der Öffnungsverschließeinrichtung interpretierbar.

Bevorzugt sind die Magneteinrichtung und die Erfassungseinrichtung feststehend angeordnet.

Bevorzugt stehen die Magneteinrichtung und die Erfassungseinrichtung derart in Wirkverbindung, dass die Erfassungseinrichtung einen Sensor ausbildet, der dazu konfiguriert ist, permanent durch die Magneteinrichtung geschaltet zu werden und seine Signalausgabe erst dann zu verändern, wenn die Störkomponente in die Luftstrecke einschwenkt und das Magnetfeld der Magneteinrichtung zumindest abschwächt oder unterbricht.

Bevorzugt bestehen gehäuseseitig umgebende Komponenten einschließlich aller Gehäuseteile aus einem nicht ferromagnetischen Werkstoff.

Bevorzugt bestehen gehäuseseitig umgebende Komponenten, einschließlich aller Gehäuseteile, aus Aluminium.

Bevorzugt besteht die Störkomponente aus einem ferromagnetischen Werkstoff.

Bevorzugt sind die Magneteinrichtung und die Erfassungseinrichtung jeweils an einem ersten Bauteil oder Abschnitt in einem festen Abstand zueinander angeordnet, und ist die Störkomponente an einem zweiten Bauteil oder Abschnitt angeordnet, das/der sich von dem ersten Bauteil oder Abschnitt unterscheidet.

Bevorzugt bilden die Erfassungseinrichtung und die Magneteinrichtung eine Reedsensor / Magnet-Paarung aus.

Alternativ bevorzugt bilden die Erfassungseinrichtung und die Magneteinrichtung eine Hallsensor / Magnet-Paarung aus.

Bevorzugt ist die Störkomponente an einem Bauteil oder Abschnitt feststehend angeordnet und ist eine die Erfassungseinrichtung und die Magneteinrichtung beinhaltende Einheit zu der Störkomponente führbar, schwenkbar und/oder drehbar.

Bevorzugt sind die Magneteinrichtung und die Erfassungseinrichtung einander gegenüberliegend angeordnet und ist zwischen ihnen als Luftstrecke ein Luftspalt einer vorbestimmten Breite ausgebildet, die ein Ein- und Ausschwenken der Störkomponente erlaubt.

Alternativ bevorzugt sind die Magneteinrichtung und die Erfassungseinrichtung auf einer gleichen Seite liegend zueinander beabstandet angeordnet und ist zwischen ihnen die Luftstrecke ausgebildet, entlang welcher die Störkomponente derart heranführbar ist, dass eine einen Schaltwechsel der Erfassungseinrichtung auslösende Umlenkung und/oder Schwächung des Magnetfelds der Magneteinrichtung eintritt.

Bevorzugt kann eine Blutbehandlungsvorrichtung eine Verriegelungszustand-Erfassungsvorrichtung der vorgenannten Art beinhalten.

Bevorzugt ist eine solche Blutbehandlungsvorrichtung als ein Dialysegerät mit zumindest einem Dialysierflüssigkeitsfilter und zumindest einem Leckagesensor als Einrichtungen zur Überwachung des zumindest einen Dialysierflüssigkeitsfilters ausgebildet.
Die Erfindung wird nachstehend anhand bevorzugter Ausführungsbeispiele unter Bezugnahme auf die beigefügte Zeichnung näher beschrieben. Es zeigen:
Fig. 1 schematisch, ausschnittsweise und stark vereinfacht eine Ansicht, die eine Verriegelungszustand-Erfassungsvorrichtung für eine Tür oder Klappe mit einer Riegelerkennung gemäß einem Ausführungsbeispiel in einer Schnittansicht darstellt; und
Fig. 2 schematisch eine ausschnittsweise perspektivische Ansicht der Verriegelungszustand-Erfassungsvorrichtung nach Fig. 1 in verbautem Zustand.
Es wird angemerkt, dass gleiche Bezugszeichen in den Figuren jeweils dieselben Elemente bezeichnen und dass mehrfach gleich dargestellte Elemente grundlegend gleichwirkender Art und insoweit nicht in jedem Falle mit Bezugszeichen versehen sind. Stellvertretend ist für mehrfach gleich dargestellte Elemente jeweils ein Element bezeichnet.

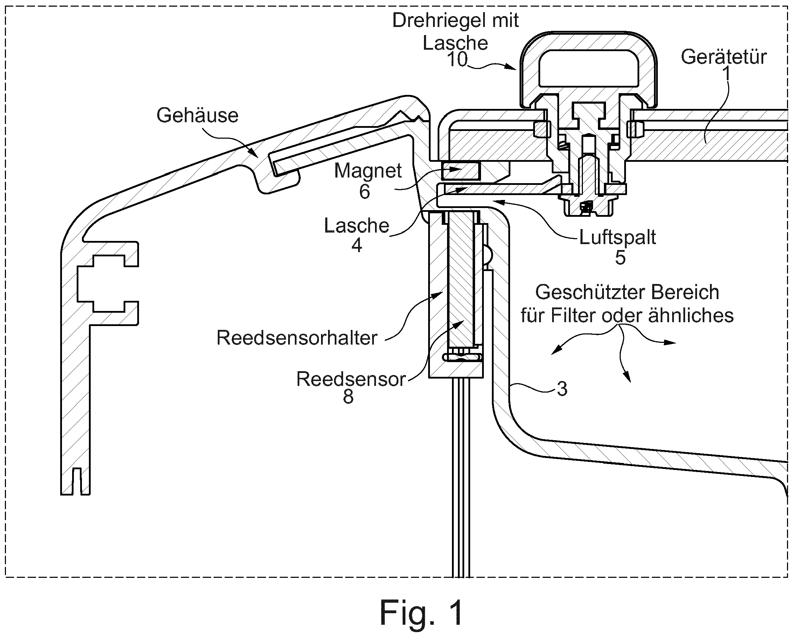

Fig. 1 zeigt schematisch, ausschnittsweise und stark vereinfacht eine Ansicht, die eine Verriegelungszustand-Erfassungsvorrichtung mit bzw. in Verbindung mit einer Verriegelungsvorrichtung mit Riegelerkennung für eine eine Öffnung eines Geräts verschließende Tür oder Klappe gemäß einem Ausführungsbeispiel in einer Schnittansicht darstellt.

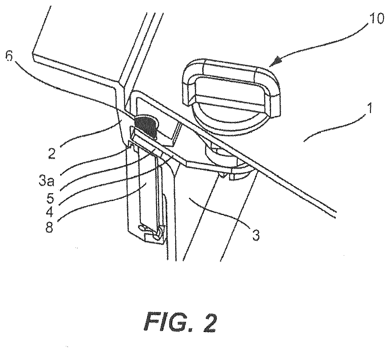

Die in Fig. 1 in einer Schnittansicht und in Fig. 2 in perspektivischer Darstellung gezeigte Verriegelungszustand-Erfassungsvorrichtung weist einen Magneten 6 als eine Magneteinrichtung auf, die an einem ersten Abschnitt 2 eines Gehäuses beispielsweise einer Blutbehandlungsvorrichtung wie etwa einer Dialysemaschine, das eine Öffnung aufweist, die mittels einer Tür 1 (oder einer Klappe) als einer Öffnungsverschließeinrichtung verschließbar ist, angeordnet ist. An einem zweiten Abschnitt 3 des Gehäuses ist als eine Erfassungseinrichtung ein Sensor 8 angeordnet, der positionell zu dem ersten Abschnitt derart beabstandet ist, dass eine Luftstrecke 5, in Fig. 1 ein Luftspalt, zwischen dem ersten und dem zweiten Abschnitt ausgebildet ist. Der Sensor 8 kann dabei verschleißgeschützt durch eine Gehäuseauskragung oder Gehäusewandung 3a abgedeckt sein, d.h. muss mit seiner Stirnseite nicht notwendigerweise in der Luftstrecke bzw. dem Luftspalt liegen. Eine Störkomponente, in diesem Ausführungsbeispiel ein Riegel 4, ist an einem in der Tür 1 angeordneten Schließ- oder Verriegelungsmechanismus 10, der eine Störkomponentenverschwenkeinrichtung bildet, verdrehfest festgelegt und dazu angeordnet, bei bzw. mit Drehung des Verriegelungsmechanismus 10 (beispielsweise im Uhrzeigersinn/gegen den Uhrzeigersinn) in die Luftstrecke 5 einzuschwenken und aus dieser auszuschwenken. Der Sensor 8 ist dazu angeordnet, in Zusammenwirkung mit dem Magneten 6 bzw. dessen Magnetfeld und dem Riegel 4 als das Magnetfeld beeinflussender (abschwächender, umlenkender und/oder unterbrechender) Störkomponente einen verriegelten Zustand und einen nicht verriegelten Zustand der Tür 1 zu erfassen und zur Weiterverarbeitung an beispielsweise nachgeschaltete Hardware und/oder Software zu signalisieren.

In anderen Worten sind der Magnet 6 und der Sensor 8 jeweils an einem ersten (Gehäuse-) Bauteil oder Abschnitt in einem festen Abstand zueinander angeordnet, und ist der drehbare Riegel 4 an einem zweiten Bauteil oder Abschnitt, in diesem Ausführungsbeispiel der Tür 1, angeordnet, die sich insoweit von dem ersten, dem Gehäuse zuzurechnenden Bauteil oder Abschnitt unterscheidet.

In diesem Ausführungsbeispiel ist der Sensor 8 dazu angeordnet, nur dann, wenn sich der Riegel 4 in einer in die Luftstrecke eingeschwenkten Position befindet, einen verriegelten Zustand der Tür 1 zu erfassen und zu signalisieren, d.h. der Sensor 8 ist dazu konfiguriert, eine Zustandserkennung dieser Gerätetür durchzuführen, wobei in einer vorbestimmten Position der Störkomponente ein tatsächlich verriegelter Zustand der Gerätetür erkannt und nur dann der Betrieb des Geräts für den Anwender freigegeben wird. In anderen Worten erkennt der Sensor nicht nur, ob bzw. dass die Tür 1 geschlossen ist, sondern darüber hinaus, dass sie auch ordnungsgemäß verriegelt ist und gibt erst bei Vorliegen des verriegelten Zustands durch sein diesem Zustand entsprechendes Ausgangssignal die Gerätefunktion frei.

Die Verschaltung des Sensors 8 ist in diesem Zusammenhang derart konfiguriert, dass sein Ausgangssignal bei beispielsweise einer Unterbrechung an Anschlussmitteln, die zumindest einen Stecker und/oder eine Kabelverbindung beinhalten, ebenfalls dem nicht verriegelten Zustand entspricht, so dass eine Fehlfunktion oder ein Fehler dieser Art erfassbar und als entriegelter Zustand der Tür (mit in der Folge Sperrung der Gerätefunktionalität) interpretierbar ist.

In dem in Fig. 1 gezeigten Beispiel sind der Magnet 6 und der Sensor 8 feststehend angeordnet, d.h. an jeweiligen Gehäuseteilen oder Gehäuseabschnitten ortsfest verbaut und stehen dabei derart miteinander in Wirkverbindung, dass der Sensor 8 permanent durch den Magneten 6 bzw. dessen Magnetfeld geschaltet wird und seine Signalausgabe erst dann verändert, wenn der Riegel 4 in die Luftstrecke einschwenkt und das Magnetfeld zumindest abschwächt oder unterbricht.

Dies funktioniert in dem vorliegenden Ausführungsbeispiel besonders gut, wenn gehäuseseitig umgebende Komponenten (beispielsweise alle Gehäuseteile) aus einem nicht ferromagnetischen Werkstoff, etwa Aluminium, bestehen, und der Riegel 4 aus einem ferromagnetischen Werkstoff besteht.

In diesem Fall bilden der Sensor 8 und der Magnet 6 eine nach dem Reedkontaktprinzip arbeitende Reedsensor / Magnet-Paarung aus. Alternativ kann vorgesehen sein, dass der Sensor 8 und der Magnet 6 eine Hallsensor / Magnet-Paarung aus, die weiter vorteilhaft eine Anpassung oder Einstellung und damit Verbesserung der Schaltgenauigkeit ermöglicht.

In einer alternativen (nicht dargestellten) Ausführungsform kann der Riegel 4 an einem vorbestimmten Bauteil oder Abschnitt feststehend angeordnet sein und können der Sensor 8 und der Magnet 6 an einer Einheit angeordnet sein, die zu der Störkomponente führbar, schwenkbar und/oder drehbar ist. In anderen Worten kann beispielsweise der Riegel 4 oder ein zungenförmiges Element an dem Gehäuseabschnitt 2 oder 3 fest angeordnet sein und kann eine aus Sensor 8 und Magnet 6 in vorbestimmter positioneller Zuordnung bestehende Einheit oder Baugruppe an der Verriegelungsvorrichtung 10 festgelegt sein und bei deren Rotation so an den feststehenden Riegel 4 geschwenkt werden, dass dieser in der Luftstrecke bzw. dem Luftspalt 5 der Sensor-Magnet-Anordnung zu liegen kommt. Es wird angemerkt, dass für den Fall eines Luftspalts der Magnet 6 und der Sensor 8 einander gegenüberliegend angeordnet sind und zwischen ihnen als Luftstrecke der Luftspalt einer vorbestimmten Breite ausgebildet ist, die ein Ein- und Ausschwenken des Riegels 4 erlaubt.

Alternativ können der Magnet 6 und der Sensor 8 auf ein und derselben, d.h. einer gleichen, Seite liegend zueinander beabstandet angeordnet sein. In diesem Fall ist zwischen diesen die Luftstrecke ausgebildet, entlang welcher der Riegel 4 derart an Sensor 8 und Magnet 6 heranführbar ist, dass eine einen Schaltwechsel des Sensors 8 auslösende Umlenkung und/oder Schwächung des Magnetfelds des Magneten 6 herbeigeführt wird.

Ein speziell vorteilhafter Anwendungsbereich des vorliegenden Ausführungsbeispiels liegt in einer extrakorporalen Blutbehandlungsvorrichtung, die eine Verriegelungszustand-Erfassungsvorrichtung wie vorstehend beschrieben beinhaltet. Insbesondere kann es sich bei einer solchen Blutbehandlungsvorrichtung um ein Dialysegerät mit zumindest einem Dialysierflüssigkeitsfilter und zumindest einem Leckagesensor als eine von Einrichtungen zur Überwachung des zumindest einen Dialysierflüssigkeitsfilters handeln.

Die Erfindung ist allerdings nicht auf extrakorporale Blutbehandlungsvorrichtungen beschränkt, sondern ist speziell auf dem Gebiet der Medizin dort anwendbar, wo eine sichere Verriegelung einer Tür oder Klappe sicherzustellen ist und eine nicht sicher verriegelte Tür oder Klappe aus Sicherheitsgründen zu einer Zwangsabschaltung oder zwangsweisen Außerbetriebsetzung einer die der Erfassung unterliegende Tür oder Klappe beinhaltenden Einrichtung oder Vorrichtung bzw. eines entsprechenden Systems, einer entsprechenden Anlage und dergleichen führen soll.

Die Erfindung leistet danach vorteilhaft eine sichere Erkennung eines Öffnens einer Tür oder einer nicht korrekt verschlossenen Tür durch einen Sensor. Die relevanten Vorrichtungsteile einschließlich nachgeschalteter Verarbeitung (Hardware und Software) sind unabhängig von der Umgebungstemperatur, da der Sensor zwischen genau zwei Zuständen schaltet, die auch bei wechselnden Umgebungstemperaturen sicher erreichbar sind. Eine Beeinträchtigung der Funktion durch Verschmutzung ist aufgrund der geschützt möglichen Positionierung am Gerät und dem berührungslosen Schaltvorgang ausgeschlossen. Sowohl Magnet als auch Sensor sind wartungsfrei feststehend in einem Bauteil oder Gehäuse unterbringbar und verbaubar. Sämtliche Bauteile einschließlich des Gehäuses sind aus allen nicht ferromagnetischen Werkstoffen einschließlich Kunststoffen und/oder Aluminium fertigbar. Es wird keinerlei zusätzliche Spannungsversorgung an der Verriegelungszustand-Erfassungsvorrichtung benötigt und die gesamte Anordnung ist frei von Beeinflussung durch Umgebungslicht. Teure und empfindliche Komponenten wie beispielsweise Sender und/oder Empfänger sind nicht vonnöten. Außerdem wird ein Kabelbruch oder ein nicht gesteckter Stecker von dem Gerät erkannt, was dann sofort den sicheren Zustand herstellen kann.

Insgesamt stellt die erfindungsgemäße Schaltung eine Erkennung einer verriegelten Tür bereit und kann verhindern, dass ein versehentliches Öffnen unbemerkt bleibt. Des Weiteren ist die vorgeschlagene Lösung sicher in der Ausführung, d.h. das System wird durch fertigungsbedingte Streuung der Magnetfelder bzw. unterschiedliche Schalthysteresen des Sensors nicht beeinträchtigt. Das System kann auch nicht durch Störeinflüsse von außen (Verschmutzung, Temperaturwechsel, unterschiedliche Lichtverhältnisse, Verschleiß und dergleichen) beeinträchtigt werden.

Wie vorstehend beschrieben wurde, beinhaltet eine Verriegelungszustand-Erfassungsvorrichtung eine Magneteinrichtung 6 (in dem vorstehend beschriebenen Ausführungsbeispiel ein Magnet), die an einem ersten Abschnitt eines Gerätegehäuses angeordnet ist, das eine Öffnung aufweist, die mittels einer Öffnungsverschließeinrichtung (in dem vorstehend beschriebenen Ausführungsbeispiel eine Tür oder eine Klappe) verschließbar ist. Eine Erfassungseinrichtung 8 (in dem vorstehend beschriebenen Ausführungsbeispiel ein Sensor) ist an einem zweiten Abschnitt des Gehäuses angeordnet, der positionell zu dem ersten Abschnitt derart beabstandet ist, dass eine Luftstrecke zwischen dem ersten und dem zweiten Abschnitt ausgebildet ist. Eine Störkomponente 4 (in dem vorstehend beschriebenen Ausführungsbeispiel ein schwenkbarer Riegel) ist an einer in der Öffnungsverschließeinrichtung angeordneten Störkomponentenverschwenkeinrichtung (in dem vorstehend beschriebenen Ausführungsbeispiel eine Anordnung, mittels welcher der Riegel beispielsweise zwischen vorbestimmten Positionen hin und her bewegt werden kann) verdrehfest festgelegt und dazu angeordnet, mit Drehung der Störkomponentenverschwenkeinrichtung in die Luftstrecke einzuschwenken und aus dieser auszuschwenken. Die Erfassungseinrichtung 8 ist dazu angeordnet, in Zusammenwirkung mit der Magneteinrichtung 6 und der Störkomponente 4 einen verriegelten Zustand und einen nicht verriegelten Zustand der Öffnungsverschließeinrichtung zu erfassen und zur Weiterverarbeitung zu signalisieren.

## Patentansprüche

1. Medizinisches Gerät, das eine Verriegelungszustand-Erfassungsvorrichtung aufweist mit:
einer Magneteinrichtung (6), die an einem ersten Abschnitt (2) eines Gehäuses angeordnet ist, das eine Öffnung aufweist, die mittels einer Öffnungsverschließeinrichtung (1) verschließbar ist;
einer Erfassungseinrichtung (8), die an einem zweiten Abschnitt (3) des Gehäuses angeordnet ist, der positionell zu dem ersten Abschnitt (2) derart beabstandet ist, dass eine Luftstrecke (5) zwischen dem ersten und dem zweiten Abschnitt (2, 3) ausgebildet ist; und
einer Störkomponente (4), die an einer in der Öffnungsverschließeinrichtung (1) angeordneten Störkomponentenverschwenkeinrichtung (10) verdrehfest festgelegt und dazu angeordnet ist, mit Drehung der Störkomponentenverschwenkeinrichtung (10) in die Luftstrecke (5) einzuschwenken und aus dieser auszuschwenken,
wobei die Erfassungseinrichtung (8) dazu angeordnet ist, in Zusammenwirkung mit der Magneteinrichtung (6) und der Störkomponente (4) einen verriegelten Zustand und einen nicht verriegelten Zustand der Öffnungsverschließeinrichtung (1) zu erfassen und zur Weiterverarbeitung zu signalisieren.

2. Medizinisches Gerät nach Anspruch 1, bei der die Erfassungseinrichtung (8) dazu angeordnet ist, nur dann, wenn sich die Störkomponente (4) in einer in die Luftstrecke (5) eingeschwenkten Position befindet, einen verriegelten Zustand der Öffnungsverschließeinrichtung (1) zu erfassen und zu signalisieren.

3. Medizinisches Gerät nach Anspruch 1 oder 2, bei der die Öffnungsverschließeinrichtung (1) eine Tür oder eine Klappe ist und die Erfassungsvorrichtung (8) dazu konfiguriert ist, eine Zustandserkennung dieser Tür oder Klappe durchzuführen, wobei in einer vorbestimmten Position der Störkomponente (4) ein verriegelter Zustand der Tür oder Klappe erkannt und nur dann der Betrieb des Geräts für den Anwender freigegeben wird.

4. Medizinisches Gerät nach einem der vorangehenden Ansprüche, bei der eine Unterbrechung an Anschlussmitteln, die zumindest einen Stecker und/oder eine Kabelverbindung beinhalten, erfassbar und als entriegelter Zustand der Öffnungsverschließeinrichtung (1) interpretierbar ist.

5. Medizinisches Gerät nach einem der vorangehenden Ansprüche, bei der die Magneteinrichtung (6) und die Erfassungseinrichtung (8) feststehend angeordnet sind.

6. Medizinisches Gerät nach einem der vorangehenden Ansprüche, bei der die Magneteinrichtung (6) und die Erfassungseinrichtung (8) derart in Wirkverbindung stehen, dass die Erfassungseinrichtung (8) einen Sensor ausbildet, der dazu konfiguriert ist, permanent durch die Magneteinrichtung (6) geschaltet zu werden und seine Signalausgabe erst dann zu verändern, wenn die Störkomponente (4) in die Luftstrecke (5) einschwenkt und das Magnetfeld der Magneteinrichtung (6) zumindest abschwächt oder unterbricht.

7. Medizinisches Gerät nach einem der vorangehenden Ansprüche, bei der gehäuseseitig umgebende Komponenten einschließlich aller Gehäuseteile aus einem nicht ferromagnetischen Werkstoff bestehen.

8. Medizinisches Gerät nach einem der vorangehenden Ansprüche, bei der gehäuseseitig umgebende Komponenten, einschließlich aller Gehäuseteile, aus Aluminium und/oder Kunststoff bestehen.

9. Medizinisches Gerät nach einem der vorangehenden Ansprüche, bei der die Störkomponente (4) aus einem ferromagnetischen Werkstoff besteht.

10. Medizinisches Gerät nach einem der vorangehenden Ansprüche, bei der die Magneteinrichtung (6) und die Erfassungseinrichtung (8) jeweils an einem ersten Bauteil oder Abschnitt in einem festen Abstand zueinander angeordnet sind und die Störkomponente (4) an einem zweiten Bauteil oder Abschnitt angeordnet ist, das/der sich von dem ersten Bauteil oder Abschnitt unterscheidet.

11. Medizinisches Gerät nach einem der vorangehenden Ansprüche, bei der die Erfassungseinrichtung (8) und die Magneteinrichtung (6) eine Reedsensor / Magnet-Paarung ausbilden.

12. Medizinisches Gerät nach einem der Ansprüche 1 bis 10, bei der die Erfassungseinrichtung (8) und die Magneteinrichtung (6) eine Hallsensor / Magnet-Paarung ausbilden.

13. Medizinisches Gerät nach einem der Ansprüche 1 bis 12, bei der die Störkomponente (4) an einem Bauteil oder Abschnitt feststehend angeordnet und eine die Erfassungseinrichtung (8) und die Magneteinrichtung (6) beinhaltende Einheit zu der Störkomponente (4) führbar, schwenkbar und/oder drehbar ist.

14. Medizinisches Gerät nach einem der Ansprüche 1 bis 13, bei der die Magneteinrichtung (6) und die Erfassungseinrichtung (8) einander gegenüberliegend angeordnet sind und zwischen ihnen als die Luftstrecke (5) ein Luftspalt einer vorbestimmten Breite ausgebildet ist, die ein Ein- und Ausschwenken der Störkomponente (4) in und aus dem Spalt erlaubt.

15. Medizinisches Gerät nach einem der Ansprüche 1 bis 13, bei der die Magneteinrichtung (6) und die Erfassungseinrichtung (8) auf einer gleichen Seite liegend zueinander beabstandet angeordnet sind und zwischen ihnen die Luftstrecke (5) ausgebildet ist, entlang welcher die Störkomponente (4) derart heranführbar ist, dass eine einen Schaltwechsel der Erfassungseinrichtung (8) auslösende Umlenkung und/oder Schwächung des Magnetfelds der Magneteinrichtung (6) eintritt.

16. Medizinisches Gerät nach einem der vorangehenden Ansprüche 1 bis 15, wobei das Medizinische Gerät eine Extrakorporale Blutbehandlungsvorrichtung ist.

17. Medizinisches Gerät nach Anspruch 16, das als ein Dialysegerät mit zumindest einem Dialysierflüssigkeitsfilter und zumindest einem Leckagesensor als Einrichtungen zur Überwachung des zumindest einen Dialysierflüssigkeitsfilters ausgebildet ist.

## Claims

1. A medical device comprising a lock state detection device, comprising:
a magnet (6) disposed at a first portion (2) of a casing which has an opening that is lockable by means of an opening locking means (1);
a detector (8) disposed at a second portion (3) of the casing, the second portion being spaced apart from the first portion (2) such that a clearance (5) between the first and second portions (2, 3) is formed; and
an interfering component (4) which is fixed to an interfering component pivoting means (10) disposed in the opening locking means (1) in a non-pivotable manner and arranged to pivot into the clearance (5) and out of the clearance (5) when the interfering component pivoting means (10) is rotated, wherein
the detector (8) is arranged to detect a locked state and a non-locked state of the opening locking means (1) in cooperation with the magnet (6) and the interfering component (4), and to indicate the state for subsequent processing.

2. A medical device as defined in claim 1, wherein the detector (8) is arranged to detect and to signal a locked state of the opening locking means (1) only when the interfering component (4) is located at a position within the clearance (5).

3. A medical device according to claim 1 or 2, wherein the opening locking means (1) is a door or a lid and the detector (8) is configured to perform a recognition of a state of this door or lid, wherein a locked state of the door or lid is recognized at a predetermined position of the interfering component (4) and only then the operation of the device is allowed for an operator.

4. A medical device according to one of the preceding claims, wherein an interruption at connection means, which include at least a connector and/or a wiring connection, is detectable and interpretable as an unlocked state of the opening locking means (1).

5. A medical device according to one of the preceding claims, wherein the magnet (6) and the detector (8) are fixedly arranged.

6. A medical device according to one of the preceding claims, wherein the magnet (6) and the detector (8) are in operative connection such that the detector (8) forms a sensor configured to be permanently switched by the magnet (6) and to change its signal output only when the interfering component (4) moves into the clearance (5) and interrupts or at least weakens the magnetic field of the magnet (6).

7. A medical device according to one of the preceding claims, wherein casing-side peripheral components including all casing parts are made of a non-ferromagnetic material.

8. A medical device according to one of the preceding claims, wherein casing-side peripheral components including all casing parts are made of aluminum and/or plastic material.

9. A medical device according to one of the preceding claims, wherein the interfering component (4) is made of a ferromagnetic material.

10. A medical device according to one of the preceding claims, wherein the magnet (6) and the detector (8) are respectively arranged at a first component or portion at a fixed distance from each other, and the interfering component (4) is arranged at a second component or portion which is different from the first component or portion.

11. A medical device according to one of the preceding claims, wherein the detector (8) and the magnet (6) form a reed sensor/magnet combination.

12. A medical device according to one of claims 1 to 10, wherein the detector (8) and the magnet (6) form a hall effect sensor/magnet combination.

13. A medical device according to one of claims 1 to 12, wherein the interfering component (4) is fixedly arranged at a component or portion, and a unit including the detector (8) and the magnet (6) is movable, pivotable and/or rotatable to the interfering component (4).

14. A medical device according to one of claims 1 to 13, wherein the magnet (6) and the detector (8) are disposed facing each other, and an air gap of a predetermined width is formed between them as the clearance (5), the predetermined width allowing a movement of the interfering component (4) into and out of the gap.

15. A medical device according to one of claims 1 to 13, wherein the magnet (6) and the detector (8) are disposed at a same side and spaced from one another, and the clearance (5) is formed between them, along which the interfering component (4) is approachable such that a deviation and/or weakening of the magnetic field of the magnet (6) occurs that triggers a switchover of the detector (8).

16. A medical device according to one of preceding claims 1 to 15, wherein the medical device comprises an extracorporeal blood treatment device.

17. A medical device according to claim 16, configured as a dialysis machine having at least one dialysis liquid filter and at least one leakage sensor as means for monitoring the at least one dialysis liquid filter.

## Revendications

1. Appareil médical, qui présente un dispositif de détection d'état de verrouillage, avec :
un système magnétique (6), qui est disposé au niveau d'une première section (2) d'un boîtier, qui présente une ouverture, qui peut être fermée au moyen d'un système de fermeture d'ouverture (1) ;
un système de détection (8), qui est disposé au niveau d'une deuxième section (3) du boîtier, qui est positionnée à une distance par rapport à la première section (2) telle qu'un trajet d'air (5) est réalisé entre la première et la deuxième section (2, 3) ; et
une composante parasite (4), qui est fixée de manière solidaire en rotation au niveau d'un système de pivotement de composante parasite (10) disposé dans le système de fermeture d'ouverture (1) et est disposée pour rentrer par pivotement dans le trajet d'air (5) et en sortir par pivotement avec la rotation du système de pivotement de composante parasite (10),
dans lequel le système de détection (8) est disposé pour détecter, en coopération avec le système magnétique (6) et la composante parasite (4), un état verrouillé et un état non verrouillé du système de fermeture d'ouverture (1) et le signaler aux fins du traitement ultérieur.

2. Appareil médical selon la revendication 1, dans lequel le système de détection (8) est disposé pour détecter et signaler un état verrouillé du système de fermeture d'ouverture (1) seulement quand la composante parasite (4) se trouve dans une position rentrée par pivotement dans le trajet d'air (5).

3. Appareil médical selon la revendication 1 ou 2, dans lequel le système de fermeture d'ouverture (1) est une porte ou une trappe, et le dispositif de détection (8) est configuré pour réaliser une identification d'état de ladite porte ou trappe, dans lequel un état verrouillé de la porte ou de la trappe est identifié dans une position prédéfinie de la composante parasite (4) et seulement alors le fonctionnement de l'appareil est débloqué pour l'utilisateur.

4. Appareil médical selon l'une quelconque des revendications précédentes, dans lequel une coupure peut être détectée au niveau de moyens de raccordement, qui renferment au moins un connecteur et/ou une liaison par câble et peut être interprétée en tant qu'état déverrouillé du système de fermeture d'ouverture (1).

5. Appareil médical selon l'une quelconque des revendications précédentes, dans lequel le système magnétique (6) et le système de détection (8) sont disposés de manière immobile.

6. Appareil médical selon l'une quelconque des revendications précédentes, dans lequel le système magnétique (6) et le système de détection (8) coopèrent de telle manière que le système de détection (8) réalise un capteur, qui est configuré pour être activé de manière permanente par le système magnétique (6) et modifier son émission de signal seulement quand la composante parasite (4) rentre par pivotement dans le trajet d'air (5) et au moins affaiblit ou coupe le champ magnétique du système magnétique (6).

7. Appareil médical selon l'une quelconque des revendications précédentes, dans lequel des composantes environnantes côté boîtier y compris toutes les parties de boîtier sont constituées d'un matériau non ferromagnétique.

8. Appareil médical selon l'une quelconque des revendications précédentes, dans lequel des composantes environnantes côté boîtier y compris toutes les parties de boîtier sont constituées d'aluminium et/ou de matière plastique.

9. Appareil médical selon l'une quelconque des revendications précédentes, dans lequel la composante parasite (4) est constituée d'un matériau ferromagnétique.

10. Appareil médical selon l'une quelconque des revendications précédentes, dans lequel le système magnétique (6) et le système de détection (8) sont disposés à une distance fixe l'un de l'autre respectivement au niveau d'un premier composant ou d'une première section, et la composante parasite (4) est disposée au niveau d'un deuxième composant ou d'une deuxième section, qui se distingue du premier composant ou de la première section.

11. Appareil médical selon l'une quelconque des revendications précédentes, dans lequel le système de détection (8) et le système magnétique (6) réalisent un capteur Reed/un appariement magnétique.

12. Appareil médical selon l'une quelconque des revendications 1 à 10, dans lequel le système de détection (8) et le système magnétique (6) réalisent un capteur à effet Hall/un appariement magnétique.

13. Appareil médical selon l'une quelconque des revendications 1 à 12, dans lequel la composante parasite (4) est disposée de manière immobile au niveau d'un composant ou d'une section et une unité renfermant le système de détection (8) et le système magnétique (6) peut être guidée, pivotée et/ou tournée par rapport à la composante parasite (4).

14. Appareil médical selon l'une quelconque des revendications 1 à 13, dans lequel le système magnétique (6) et le système de détection (8) sont disposés de manière à se faire face l'un l'autre, et un entrefer d'une largeur prédéfinie est réalisé entre ces derniers en tant que trajet d'air (5), qui permet une entrée et sortie par pivotement de la composante parasite (4) dans et hors de la fente.

15. Appareil médical selon l'une quelconque des revendications 1 à 13, dans lequel le système magnétique (6) et le système de détection (8) sont disposés de manière espacée l'un de l'autre sur un même côté, et le trajet d'air (5) est réalisé entre eux, le long duquel la composante parasite (4) peut être guidée et rapprochée de telle manière qu'une déviation déclenchant un changement de commutation du système de détection (8) et/ou un affaiblissement du champ magnétique du système magnétique (6) ont lieu.

16. Appareil médical selon l'une quelconque des revendications précédentes 1 à 15, dans lequel l'appareil médical est un dispositif de traitement du sang extracorporel.

17. Appareil médical selon la revendication 16, qui est réalisé sous la forme d'un appareil de dialyse avec au moins un filtre de liquide de dialyse et au moins un capteur de fuite en tant que systèmes servant à surveiller l'au moins un filtre de liquide de dialyse.
